# EUROPEAN PATENT APPLICATION

(11) **EP 3 747 452 A1**
(43) Date of publication of application: **09.12.2020**
(21) Application number: 19747020.6
(22) Date of filing: 31.01.2019
(51) Int. Cl.: A61K 36/185, A23L 33/105, A23K 10/30

(54) **COMPOSITION FOR IMPROVING PHYSICAL STRENGTH OR ATHLETIC ABILITY, CONTAINING NUTMEG EXTRACT AS ACTIVE INGREDIENT**

(30) Priority: 01.02.2018 KR 20180012986
(71) Applicant: Korea Basic Science Institute, Daejeon 34133 (KR)
(72) Inventor: JANG, Ik Soon, Daejeon 34133 (KR); CHOI, Jong Soon, Daejeon 34133 (KR); JANG, Hyun Jin, Daejeon 34133 (KR)
(74) Representative: Avidity IP
(86) International application number: PCT/KR2019/001397
(87) International publication number: WO 2019/151811

(57) **Abstract**

The present invention relates to a composition for improving physical strength or athletic ability, containing a nutmeg extract as an active ingredient and, more specifically, to a composition for improving physical strength or athletic ability, for treating sarcopenia, for improving muscle function, and for anti-fatigue, containing a nutmeg extract as an active ingredient.

The nutmeg extract of the present invention contains a high quantity of ligand-based compounds and exhibits, when taken, very excellent effects of improving athletic ability, improving endurance and improving muscular strength, and thus is effective for treating sarcopenia and can be effectively used in medical supplies, food and the like for improving physical strength or athletic ability.

## Description

### [THECHNICAL FIELD]

This application claims priority from Korean Patent Application No. 10-2018-0012986, filed on February 01, 2018, the entire contents of which are incorporated herein by reference.

The present invention relates to a composition for improving physical strength or athletic ability, containing a nutmeg extract as an active ingredient and, more specifically, to a composition for improving physical strength or athletic ability, for treating sarcopenia, for improving muscle function, and for anti-fatigue, containing a nutmeg extract as an active ingredient.

### [BACKGROUND OF THE INVENTION]

Even though modern people pay tremendous attention to beauty and health, they suffer from obesity, hypertension, and various other adult diseases, and despite many efforts to prevent aging, they are unable to prevent it due to air pollution and heavy stress. In order to prevent such various adult diseases and aging, steady exercise is the most effective and economical, but since the time to exercise is limited, various preparations to increase the effect of exercise are commercially available. In addition, athletes have combined scientific training, diet and ergogenic aids of athletic ability for each sport to set a new record. Among them, supplements to improve athletic ability not only improve athletic performance, but are also commonly used by the general public because they are effective in removing fatigue elements that accumulate in the body and cause fatigue during physical activity. Research related to functional supplements to improve athletic ability has been actively studied, regardless of East and West. Most of the products on the market today contain steroids, caffeine, sodium hydrogen carbonate and sodium citrate. However, the effectiveness of these ingredients is only a temporary phenomenon and becomes resistant, leading to the search for products containing more and more ingredients, which can have fatal side effects to health.

Muscle (Muscle) is the most constitutive tissue in the human body to maintain the functional capacity of the human body, and to prevent metabolic diseases, it is essential to secure an adequate muscle mass. Muscle size is regulated by intracellular signaling pathways that induce anabolic or catabolism in the muscles. When a signal transduction reaction that induces synthesis occurs more than the degradation of muscle protein, muscle protein synthesis increases, and as a result, muscle hypertrophy or muscle hyperplasia increases, which increases muscle size.

Skeletal muscles are the largest organs in the human body, accounting for 40-50% of the total weight. It plays an important role in various metabolic functions in the body, including energy homeostasis and heat generation. A person's muscles decrease by more than 1% every year since the age of 40, and by the age of 80, 50% of the maximum muscle mass decreases, and muscle loss in old age is recognized as the most important factor that decreases overall physical function. As aging progresses, the body shape such as muscle and fat content, skeletal distortion, etc. are changed, and the prevalence of obesity due to muscle reduction in old age has been increasing continuously at the level of over 30% worldwide. In the case of insulin secretion or more, it may cause muscle development disorder by failing to properly supply energy to cells. Muscle loss can also be caused by various chronic diseases and aging processes. As the body ages, part of the newly created skeletal muscle is replaced by fibrous tissue. Therefore, normal aging in humans is associated with a progressive decrease in skeletal muscle mass and strength, a condition called sarcopenia, which causes weakness and degeneration. In addition, age-related diseases such as hypertension, impaired glucose tolerance and diabetes, obesity, dyslipidemia, atherosclerosis and cardiovascular disease are also associated with loss of muscle mass. In addition, other diseases, such as cancer, autoimmune disease, infectious disease, HIV infection, AIDS, chronic inflammation, arthritis, malnutrition, kidney disease, chronic obstructive pulmonary disease (COPD), emphysema, rickets, chronic lower spine pain, peripheral nerve damage, spinal cord injury, chemical damage and central nervous system (CNS) damage, can also be associated with or cause muscle loss. Finally, conditions that cause muscle loss can result from disuse conditions, such as organ immobilization due to disease, or helplessness such as long-term wheelchair use, long-term bed care, fractures or trauma. Postoperative bed rest is estimated to result in a loss of skeletal muscle mass of about 10% per week.

In addition, decreased muscles increase arthritis, back pain and chronic pain. It can also worsen the symptoms of incontinence due to abdominal obesity, and injury due to fracture can lead to death by increasing depression in old age. Because of this, muscular dystrophy in old age is a major cause of deteriorating quality of life in connection with various diseases. It is known that sarcopenia is also closely related to senile chronic diseases such as osteoporosis, insulin resistance, and arthritis, and it is possible to inhibit a decrease in physical activity due to aging through preventing or improving sarcopenia. The world's progressive ataxia and muscle weakness treatment market was estimated at about $14 billion in 2011, and is expected to grow to an annual average compound growth rate of 9.4% after that, reaching about $23.5 billion in 2017. As the treatment method for sarcopenia, increased mitochondrial production, inhibition of muscle protein degradation and anti-inflammatory drugs have been suggested, but there is no explicit treatment.

In addition, it has been shown that 25-30 g of high quality protein should be consumed for each meal as a dietary method proposed to prevent muscular dystrophy in the elderly. This means that 4-5 eggs or about 120 g of chicken breast should be consumed at every meal, and it is difficult for the ordinary people who live in daily life to practice. Therefore, although many people choose protein supplements as an alternative in recent years, protein supplements are the cause of excessive protein intake, so there is a high possibility of side effects.

Nutmeg ( 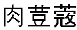 , English: Nutmeg) is a dicotyledonous Magnoliales Myristicaceae. As a tree of evergreen and broad-leaved trees, the scientific name is Miristica fragrans. Nutmeg (Myristica fragrans Houtt. or Nutmeg) fruits are used as herbal medicines and spices for the treatment of abdominal pain, diarrhea, oral mucosal diseases, joint pain, and insomnia. In addition, nutmeg fruits have been reported to have various pharmacological activities such as anti-inflammatory, antibacterial, analgesic, anti-anxiety, liver function improvement, and anti-mutation (Ozaki et al., Jpn J Pharmacol 1989; Olajide et al., Phytother Res 1999; Ram et al., J Ethnopharmacol, 1996; Sonavane et al., Pharmacol Biochem Behav 2002; Bhamarapravati et al., In Vivo, 2003; Akinboro et al., Acta Biochim Pol 2014). However, to date, there are no reports that nutmeg can be effectively used to improve athletic ability, endurance, and strength.

Accordingly, the present inventors continued to make efforts to develop a new agent improving athletic ability, and found that nutmeg extract improves the exercise ability, muscle strength, and endurance of the mouse, thereby completing the present invention.

### [DETAILED DESCRIPTION OF THE INVENTION]

### [TECHNICAL PROBLEM]

Accordingly, the present inventors were studying to find a safe natural material showing an effect of improving physical strength or athletic ability, and prepared a nutmeg extract of the present invention. They have completed the present invention after they have confirmed that the extract has a remarkable effect of increasing muscle strength and improving endurance.

Therefore, an aspect of the present invention is to provide a composition for improving physical strength or athletic ability comprising a nutmeg extract as an active ingredient.

In addition, it is to provide a composition for improving physical strength or athletic ability consisting of a nutmeg extract as an active ingredient.

In addition, it is to provide a composition for improving physical strength or athletic ability consisting essentially of a nutmeg extract as an active ingredient.

Another aspect of the present invention is to provide a composition for preventing or treating sarcopenia comprising a nutmeg extract as an active ingredient.

Also, it is to provide a composition for preventing or treating sarcopenia consisting of a nutmeg extract as an active ingredient.

In addition, it is to provide a composition for preventing or treating sarcopenia consisting essentially of a nutmeg extract as an active ingredient.

Another aspect of the present invention is to provide a composition for improving muscle function comprising a nutmeg extract as an active ingredient.

In addition, it is to provide a composition for improving muscle function consisting of a nutmeg extract as an active ingredient.

In addition, it is to provide a composition for improving muscle function consisting essentially of a nutmeg extract as an active ingredient.

Another aspect of the present invention is to provide an anti-fatigue composition comprising a nutmeg extract as an active ingredient.

Also, it is to provide an anti-fatigue composition consisting of a nutmeg extract as an active ingredient.

In addition, it is to provide an anti-fatigue composition consisting essentially of a nutmeg extract as an active ingredient.

Still another aspect of the present invention is to provide a composition as a feed additive for improving physical strength, improving athletic ability, or increasing the amount of muscle comprising a nutmeg extract as an active ingredient.

In addition, it is to provide a composition as a feed additive for improving physical strength, improving athletic ability, or increasing the amount of muscle consisting of a nutmeg extract as an active ingredient.

In addition, it is to provide a composition as a feed additive for improving physical strength, improving athletic ability, or increasing the amount of muscle consisting essentially of a nutmeg extract as an active ingredient.

Still another aspect of the present invention is to provide an use of a nutmeg extract for preparing an agent for improving physical strength or athletic ability, an agent for preventing or treating sarcopenia, an agent for improving muscle function, or an anti-fatigue agent.

Still further aspect of the present invention is to provide a method for improving physical strength or athletic ability, a method for preventing or treating sarcopenia, a method for improving muscle function, or a method for an anti-fatigue, the method comprising administering an effective amount of a composition comprising a nutmeg extract as an active ingredient to subject in need thereof.

### [TECHNICAL SOLUTION]

An embodiment according to an aspect of the present invention provides a composition for improving physical strength or athletic ability comprising a nutmeg extract as an active ingredient.

In addition, the present invention provides a composition for improving physical strength or athletic ability consisting of a nutmeg extract as an active ingredient.

In addition, the present invention provides a composition for improving physical strength or athletic ability consisting essentially of a nutmeg extract as an active ingredient.

An embodiment according to another aspect of the present invention provides a composition for preventing or treating sarcopenia comprising a nutmeg extract as an active ingredient.

In addition, it is to provide a composition for preventing or treating sarcopenia consisting of a nutmeg extract as an active ingredient.

In addition, it is to provide a composition for preventing or treating sarcopenia consisting essentially of a nutmeg extract as an active ingredient.

An embodiment according to still another aspect of the present invention provides a composition for improving muscle function comprising a nutmeg extract as an active ingredient.

In addition, the present invention provides a composition for improving muscle function consisting of a nutmeg extract as an active ingredient.

In addition, the present invention provides a composition for improving muscle function consisting essentially of a nutmeg extract as an active ingredient.

An embodiment according to another aspect of the present invention provides a composition for anti-fatigue comprising a nutmeg extract as an active ingredient.

In addition, it is to a composition for anti-fatigue consisting of a nutmeg extract as an active ingredient.

In addition, it provides a composition for anti-fatigue consisting essentially of a nutmeg extract as an active ingredient.

An embodiment according to still another aspect of the present invention provides a composition as a feed additive for improving physical strength, improving athletic ability, or increasing the amount of muscle comprising a nutmeg extract as an active ingredient.

In addition, it provides a composition as a feed additive for improving physical strength, improving athletic ability, or increasing the amount of muscle consisting of a nutmeg extract as an active ingredient.

In addition, it provides a composition as a feed additive for improving physical strength, improving athletic ability, or increasing the amount of muscle consisting essentially of a nutmeg extract as an active ingredient.

An embodiment according to still further aspect of the present invention provides a use of a nutmeg extract for preparing an agent for improving physical strength or athletic ability, an agent for preventing or treating sarcopenia, an agent for improving muscle function, or an anti-fatigue agent..

An embodiment according to still further aspect of the present invention provides a method for improving physical strength or athletic ability, a method for preventing or treating sarcopenia, a method for improving muscle function, or a method for an anti-fatigue, the method comprising administering an effective amount of a composition comprising a nutmeg extract as an active ingredient to a subject in need thereof.

Hereinafter, the present invention will be described in detail.

Nutmeg ( 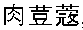 , English: Nutmeg) is a dicotyledonous Magnoliales Myristicaceae. As a tree of evergreen and broad-leaved trees, the scientific name is Miristica fragrans. The nutmeg can be used as a nutmeg prototype as it is without damage, or can be used after performing a pre-treatment process in consideration of the process speed and process (manufacturing) efficiency intended by a person skilled in the art, and the pre-treatment process includes, for example, conventional sorting, washing, cutting, powdering and drying.

Nutmeg extract of the present invention can be extracted by a known natural product extraction method. Preferably, it may be extracted with one or more solvents selected from the group consisting of water, an organic solvent having 1 to 6 carbon atoms, and a subcritical and supercritical fluid. The organic solvent having 1 to 6 carbon atoms may be selected from the group consisting of alcohol having 1 to 6 carbon atoms, acetone, ether, benzene, chloroform, ethyl acetate, methylene chloride, hexane, cyclohexane and petroleum ether, but is not limited thereto. The nutmeg extract of the present invention may be more preferably extracted with alcohol, more preferably an ethanol aqueous solution, more preferably 10 to 30% ethanol aqueous solution.

In one embodiment of the present invention, it is characterized in that an extract using nutmeg is prepared by an alcohol (ethanol) of various concentrations as a solvent. Due to this unique extraction method of the present invention, it is characterized in that it is possible to obtain the nutmeg extract with a low myristicin content. The specificity of the method for preparing the extract of the present invention is well shown in Example 1 of the specification of the present invention.

After the extraction process, filtration, centrifugation, etc. may further include a purification process to increase the purity of the extract. In addition, it may further include a concentration process. The concentration may be by a concentration device or a concentration method known in the art, but is not limited thereto. However, it may be, for example, precipitation concentration, evaporation concentration, reduced pressure concentration, a ultrafiltration method, a reverse osmosis method, a centrifugal separation method, and preferably reduced pressure concentration.

The extract of the present invention can be used as a liquid by filtration and/or concentration, and can be solidified through a common drying process such as spray drying or freeze drying. In the drying step, dextrin may be mixed and dried before spray drying or freeze drying.

The nutmeg extract of the present invention is characterized by a remarkable effect of improving athletic ability. This is well illustrated in the specification examples of the present invention. Therefore, the present invention provides a composition for improving physical strength or athletic ability comprising the nutmeg extract as an active ingredient.

The composition of the present invention is characterized by having an effect of improving physical strength or athletic ability.

"Physical strength" is a concept that includes swimming, motivation and coordination, and measures the ability and efficiency of swimming from one end of a glass tank filled with water to the escape platform on the other side in a swimming experiment.

"Athletic ability" is closely related to the performance and record improvement of athletes, and can be improved by improving cardiorespiratory function, promoting energy metabolism, restoring fatigue, and increasing muscle strength. That is, athletic ability can be improved through a method of quickly recovering accumulated fatigue during training in the long term and increasing endurance during exercise.

Therefore, the effect of improving physical strength or athletic ability of the present invention may preferably mean a muscle endurance or muscle strengthening effect.

The present invention provides a composition for preventing or treating sarcopenia comprising the nutmeg extract as an active ingredient.

In addition, the present invention provides a composition for preventing or treating sarcopenia composed of the nutmeg extract as an active ingredient.

In addition, the present invention provides a composition for preventing or treating sarcopenia consisting essentially of the nutmeg extract as an active ingredient.

In the present invention, treatment means reversing or alleviating an illness or disease to which the term applies, or one or more symptoms of the illness or disease, or inhibiting or preventing its progression. Therefore, the treatment or therapy for sarcopenia in a mammal may include one or more of the following.
(1) Reducing sarcopenia
(2) Preventing the recurrence of sarcopenia, and
(3) Palliating symptoms of sarcopenia

The present invention provides a composition for improving muscle function comprising the nutmeg extract as an active ingredient.

In addition, the present invention provides a composition for improving muscle function consisting of the nutmeg extract as an active ingredient.

In addition, the present invention provides a composition for improving muscle function consisting essentially of the nutmeg extract as an active ingredient.

In the present invention, "muscle" refers to the tendon, muscle, and tendon. "Muscle function" refers to the ability to exert power by contraction of muscles, and includes muscle power, which is the ability of a muscle to exert its maximum contractility to overcome resistance, muscle endurance, which is the ability of a muscle to show how long or how many times it can contract and relax at a given weight, and quickness, the ability to exert strong power in a short time.

The present invention provides a composition for anti-fatigue comprising the nutmeg extract as an active ingredient.

In addition, the present invention provides a composition for anti-fatigue consisting of the nutmeg extract as an active ingredient.

In addition, the present invention provides a composition for anti-fatigue consisting essentially of the nutmeg extract as an active ingredient.

The "fatigue" of the present invention is divided into mental and physical fatigue. In the present invention, "fatigue" refers to physical fatigue that accumulates in the muscle after performing exercise, which is caused by the concentration of glycogen in the muscle, the concentration of pyruvic acid, and dehydrogenation of lactate. It is caused by enzyme activity. It is described in one embodiment that the composition of the present invention reduces glycogen concentration in muscle cells and decreases creatine kinase concentration.

The effect of the nutmeg extract of the present invention is not limited to individuals and ages, but may be effective for subjects over 40 years old, more preferably over 50 years old, most preferably over 65 years old. That is, the extract of the present invention preferably may be effective for the middle-aged or older who has finished growth and aging at an early stage, and more preferably can be effective for the older or older.

As used in the present invention, the term "subject" refers to an animal or a cell or tissue of an animal, including a mammal, particularly a human.

The composition of the present invention shows effects such as improvement in athletic ability, improvement in endurance, and improvement in muscle strength regardless of age, but in particular, it is described in one embodiment that the effect on the old mouse is remarkably excellent.

The composition of the present invention may contain a nutmeg extract alone or may contain one or more active ingredients exhibiting nutmeg extract and similar functions thereof. If the additional components are included, the composition of the present invention may further enhance the effects of improving physical strength/improving athletic ability/treating sarcopenia/improving muscle function/anti-fatigue. When adding the above components, skin safety, utility of formulation, and stability of active ingredients according to the combined use may be considered.

The food composition of the present invention includes all forms of functional foods, health functionl foods, nutritional supplements, health foods, and food additives. Food compositions of this type can be prepared in various forms according to conventional methods known in the art.

For example, as a healthy food, the nutmeg extract may be prepared in the form of tea, juice, and drink for drinking or granulated, encapsulated, and powdered. In addition, the nutmeg extract of the present invention can be prepared in the form of a composition by mixing with known active ingredients known to have wrinkle improvement, anti-aging, skin elasticity enhancement and skin moisturizing effect.

In addition, functional foods can be prepared by adding the nutmeg extract to beverages (including alcoholic beverages), fruits and processed foods thereof (e.g., canned fruits, bottled food, jams, marmalades, etc.), fish, weight, and processed foods (e.g., ham, sausage corn beef, etc.), breads and noodles (e.g., udon, buckwheat noodles, ramen, spaghetti, macaroni, etc.), juice, various drinks, cookies, syrup, dairy products (e.g., butter, cheese, etc.), edible vegetable oil, margarine, vegetable protein, retort food, frozen food, and various seasonings (e.g., miso, soy sauce, sauce, etc.).

Specifically, the present invention provides a health functional food comprising the nutmeg extract and a food-pharmaceutically acceptable carrier or additive. The health functional food defined in the invention is sufficiently established in the functional and safety of the newly defined human body through the Act on Health Functional Food revised in 2008. It means that it is a health functional food that is included in the regulation on the recognition of functional raw materials for health functional food as prescribed in Notification No. 2008-72 of the Food and Drug Administration.

Such a dietary supplement may be formulated into an agent of conventional dietary supplements known in the art. The dietary supplement is, for example, powders, granules, tablets, pills, capsules, suspensions, emulsions, syrups, needles, liquids, extracts, gums, teas, jellies, or beverages can be prepared. Any carrier or additive known to be usable in the art may be used for preparing an agent to be prepared as the food-pharmaceutically acceptable carrier or additive.

The health functional food of the present invention includes various nutrients, vitamins, minerals (electrolytes), flavoring agents such as synthetic flavoring agents and natural flavoring agents, colorants and enhancers (cheese, chocolate, etc.), pectic acid and salts thereof, alginic acid, and salts thereof, organic acids, protective colloidal thickeners, pH adjusting agents, stabilizers, preservatives, glycerin, alcohols, and carbonic acid used in carbonated beverages.

In addition, it may further contain various flavoring agents or natural carbohydrates. The natural carbohydrates include conventional sugars such as monosaccharides (e.g., glucose, fructose, etc.), disaccharides (e.g., maltose, sucrose, etc.), polysaccharides (e.g., dextrin, cyclodextrin, etc.) and sugar alcohols such as xylitol, sorbitol, and erythritol. In addition, as a flavoring agent, natural flavoring agents (e.g., taumatin, stevia extract, etc.) and synthetic flavoring agents (e.g., saccharin, aspartame, etc.) may be contained.

In addition, in order to use the nutmeg extract of the present invention as a food additive, it may be prepared and used in the form of a powder or a concentrate.

The preferred content of nutmeg extract in the food composition of the present invention may be contained in the range of 0.0001 to 90% by weight, preferably 0.01 to 50% by weight relative to the total weight of the food composition.

In addition, the present invention provides a pharmaceutical composition for improving physical strength or athletic ability comprising the nutmeg extract as an active ingredient.

The pharmaceutical composition according to the present invention may contain the nutmeg extract of the present invention alone or may further contain one or more pharmaceutically acceptable carriers, excipients, or diluents. As used herein, "pharmaceutically acceptable" refers to a non-toxic composition that is physiologically acceptable, and when administered to humans, does not inhibit the action of the active ingredient and does not cause allergic reactions or similar reactions, such as gastrointestinal disorders, dizziness, or similar reactions.

Pharmaceutically acceptable carriers may further include, for example, carriers for oral administration or carriers for parenteral administration. The carriers for oral administration may include lactose, starch, cellulose derivatives, magnesium stearate and stearic acid. In addition, the carriers for parenteral administration may include water, suitable oils, saline, aqueous glucose and glycols, and further include stabilizers and preservatives. Suitable stabilizers include antioxidants such as sodium hydrogen sulfite, sodium sulfite or ascorbic acid. Suitable preservatives include benzalkonium chloride, methyl- or propyl-parabens and chlorobutanol. The pharmaceutical composition of the present invention may further include a lubricant, a humectant, a sweetener, a flavoring agent, an emulsifier and a suspension agent in addition to the above components. Other pharmaceutically acceptable carriers and agents may be referred to all known in the art.

The composition of the present invention can be administered to any mammal, including humans. For example, it can be administered orally or parenterally. The parenteral administration method is not limited thereto, but can be intravenous, intramuscular, intraarterial, intramedullary, intrathecal, intracardiac, transdermal, subcutaneous, intraperitoneal, intranasal, intestinal, topical, sublingual or rectal administration.

The pharmaceutical composition of the present invention may be formulated as an oral administration or parenteral administration preparation according to the administration route as described above. In the case of preparations for oral administration, the compositions of the present invention may be formulated using methods known in the art as powders, granules, tablets, pills, dragees, capsules, liquids, gels, syrups, slurries, suspensions, etc. For example, oral preparations can obtain tablets or dragees by blending the active ingredient with a solid excipient and then grinding it and adding a suitable adjuvant to the granule mixture. Examples of suitable excipients include sugars containing lactose, dextrose, sucrose, sorbitol, mannitol, xylitol, erythritol and maltitol, and starches containing corn starch, wheat starch, rice starch, and potato starch, and cellulose containing cellulose, methyl cellulose, sodium carboxymethylcellulose and hydroxypropylmethyl-cellulose, and fillers such as gelatin, and polyvinylpyrrolidone. In addition, in some cases, crosslinked polyvinylpyrrolidone, agar, alginic acid or sodium alginate may be added as a disintegrant. Furthermore, the pharmaceutical composition of the present invention may further include an anti-coagulant, a lubricant, a wetting agent, a flavoring agent, an emulsifying agent and a preservative.

Agents for parenteral administration may be formulated by methods known in the art in the form of injections, creams, lotions, external preprations, oils, moisturizers, gels, aerosols and nasal inhalants. These agents can include formulations generally known to all pharmaceutical chemistries.

The total effective amount of the composition of the present invention may be administered in a single dose, or may be administered by a fractionated treatment protocol in which multiple doses are administered for a long time. The pharmaceutical composition of the present invention may vary the content of the active ingredient according to the degree of disease. Preferably, the preferred total dose of the pharmaceutical composition of the present invention may be from about 0.01 µg to 1,000 mg per kg of body weight of the patient per day, most preferably from 0.1 µg to 500 mg. However, the dosage of the pharmaceutical composition is determined in consideration of various factors such as the formulation method, route of administration and frequency of treatment, as well as various factors such as the patient's age, weight, health status, sex, severity of the disease, diet and excretion rate. In view of this, those of ordinary skill in the art will be able to determine the appropriate effective dosage of the compositions of the present invention. The pharmaceutical composition according to the present invention is not particularly limited to its formulation, route of administration and method of administration as long as the effect of the present invention is shown.

The present invention provides a composition as a feed additive for improving physical strength, improving athletic ability, or increasing the amount of muscle, comprising a nutmeg extract as an active ingredient.

In addition, the present invention provides a composition as a feed additive for improving physical strength, improving athletic ability, or increasing the amount of muscle consisting of a nutmeg extract as an active ingredient.

In addition, it provides a composition as a feed additive for improving physical strength, improving athletic ability, or increasing the amount of muscle consisting essentially of a nutmeg extract as an active ingredient.

The term "feed" of the present invention means any natural or artificial food, one meal, or a component of the one meal that the animal eats. The feed containing the composition for improving physical strength or athletic ability according to the present invention as an active ingredient can be prepared in various forms of feed known in the art, and preferably, thick feed, forage and/or special feed may be included, but is not limited thereto.

In the present invention, the term "feed additive" includes a substance added to feed for various purposes such as supplementing nutrients and preventing weight loss, improving digestibility of fiber in feed, improving oil quality, preventing reproductive disorder and improving conception rate, and preventing high temperature stress in summer.

The feed additive of the present invention corresponds to the supplementary feed under the feed management method, and may further include mineral preparations such as sodium hydrogen carbonate, bentonite, magnesium oxide, and composite minerals; Mineral preparations that are trace minerals such as zinc, copper, cobalt, and selenium; Vitamin preparations such as carotene, vitamin A D, E, nicotinic acid, and vitamin B complex; Protective amino acid agents such as methionine and lysine; Protective fatty acids such as fatty acid calcium salts; Live bacteria or yeast such as probiotics (lactic acid bacteria), yeast cultures, and fungal fermentation products.

Concentrated feed stuff includes seed fruits containing cereals such as wheat, oats, and corn, bran containing rice bran, wheat bran, and barley as a byproduct of refining and obtaining grain, residues such as residual starch, is the main component of starch residue, which is the rest of starch removed from oilcake, and sweet potato and potatoes, a by-product obtained from soybean, rapeseed, sesame, flaxseed, and coco palm, fish soluble, which is a concentrate of fresh liquid obtained from fish meal, fish residue, and fish, weight powder, blood powder, milk powder, skim milk powder, cheese from milk, animal feed, such as dry whey, which is the whey that is the balance when manufacturing casein from skim milk, yeast, chlorella and seaweed, but is not limited to thereto.

Roughages include raw grasses such as wild grass, pasture, and green grass, root vegetables such as turnip for feed, beet for feed, and Luther Bearer, a kind of turnip, Silage, a storage feed that is fermented with lactic acid by filling raw grass, green grass crops, and grain chambers in silos, hay that has been dried with wild grass and pasture, crop straw for breeding, and legume leaves, but are not limited thereto.

Special feeds include mineral feeds such as oyster shells and rock salt, urea feeds such as diureid isobutane, an urea or derivative thereof, and feed additives and dietary supplements, which are substances added in a small amount to the blended feed to supplement ingredients that are likely to be insufficient when adding only the natural feedstock or to increase the storage of the feed.

The feed additive according to the present invention can be prepared by adding nutmeg extract in an appropriate effective concentration range according to various feed preparation methods known in the art.

The feed additive according to the present invention can be applied without limitation as long as it is an object aimed at improving physical strength or athletic ability and increasing weight mass. For example, it can be applied to any individual such as non-human animals such as monkeys, dogs, cats, rabbits, mormots, rats, mice, cows, sheep, pigs, goats, birds and fish.

The nutmeg extract of the present invention, but the effect is not limited to the age of the individual, preferably may be effective for the middle-aged or older who has finished growth and aging at an early stage, and more preferably can be for the older or older.

According to one example of the present invention, methanol, hot water, and ethanol extracts were prepared using crushed nutmeg.

According to another example of the present invention, as a result of comparing the body weight of the mouse group administered with ethanol extract of nutmeg, the nutmeg extract did not affect the body weight of mouse.

According to another example of the present invention, ethanol extract of nutmeg effectively increased the glycogen content of muscle cells.

According to another example of the present invention, as a result of performing a rotarod test on a mouse to which ethanol extract of nutmeg was administered, a group of mice to which nutmeg extract was administered has significantly improved exercise ability and endurance compared to a control group.

According to another example of the present invention, as a result of performing a wire hang test on mice to which the ethanol extract of nutmeg was administered, the mouse group to which the nutmeg extract was administered has the highest hanging time compared to the control group, and it was confirmed that nutmeg extract can improve athletic ability and endurance through strengthening mouse muscle strength.

According to another example of the present invention, as a result of performing a Morris water maze test on mice to which the ethanol extract of nutmeg was administered, it was confirmed that the acceptance rate of mice to which the nutmeg extract was administered was effectively improved.

According to another example of the present invention, as a result of performing a Neurological deficit test on a mouse administered with the ethanol extract of nutmeg, the number of individuals in the mouse group administered with the nutmeg extract was lower in the rotarod than the control group, so that it was confirmed that it is effective to improve the athletic ability of mouse, that is, muscle endurance.

According to another example of the present invention, as a result of measuring the muscle volume by separating the femur of the mouse to which the nutmeg extract was administered, it was confirmed that the volume of the femur was significantly increased in the nutmeg extract administration group.

According to another example of the present invention, as a result of performing the rotaro test on the mice administered with the ethanol extract of nutmeg, the mouse group administered with the nutmeg extract did not fall off the rotarod longer than the control group, so that it was confirmed that it is effective to improve the athletic ability of mouse, that is, muscle endurance.

In addition, as a result of observing by extracting the livers of normal mice and mice with long-term administration of creatine and mice with long-term administration of the ethanol extract of nutmeg, creatine induced liver toxicity, but it was confirmed that the ethanol extract of nutmeg did not cause liver toxicity.

Therefore, the composition of the present invention has an effect of improving physical strength or athletic ability by increasing muscle power and muscular endurance, and thus has a treatment for muscular dystrophy, muscle function improvement, and anti-fatigue effect. The effect of increasing muscle strength and muscle endurance does not limit the type of muscle.

The present invention provides the use of a nutmeg extract to prepare an agent for improving physical strength or athletic ability.

The present invention provides a method for improving physical strength or athletic ability in a subject, the method comprising administering an effective amount of a composition comprising a nutmeg extract as an active ingredient to the subject in need thereof.

The present invention provides a use of nutmeg extract to prepare an agent for preventing or improving sarcopenia.

The present invention provides a method for preventing or treating sarcopenia in a subject, the method comprising administering an effective amount of a composition comprising a nutmeg extract as an active ingredient to the subject in need thereof.

The present invention provides a use of nutmeg extract to prepare an agent for improving muscle function.

The present invention provides a method for improving muscle function in a subject, the method comprising administering an effective amount of a composition comprising a nutmeg extract as an active ingredient to a subject in need thereof.

The present invention provides the use of nutmeg extract for preparing an anti-fatigue agent.

The present invention provides an anti-fatigue method, the method comprising administering an effective amount of a composition comprising a nutmeg extract as an active ingredient to a subject in need thereof.

The 'effective amount' of the present invention, when administered to a subject, , refers to an amount that exhibits athletic ability, sarcopenia, muscle function, improvement of fatigue, treatment, prevention, detection, diagnosis or athletic ability, improvement of muscle function, fatigue and the inhibitory effect of the sarcopenia. The 'subject' may be an animal, preferably a mammal, particularly an animal including a human, or may be a cell, tissue, or organ derived from the animal. The subject may be a patient in need of treatment.

The 'treatment' of the present invention comprehensively refers to a decrease in athletic ability, sarcopenia, muscular dysfunction, fatigue or improvement of these symptoms, which may include treating, substantially preventing, or improving the condition of these diseases or symptoms, alleviating, treating or preventing one symptom or most symptoms resulting from decreased athletic ability, sarcopenia, muscular dysfunction, or fatigue, but is not limited thereto.

In the present invention, the term 'comprising' is used in the same way as 'containing' or 'characteristic', and does not exclude additional component elements or method steps not mentioned in the composition or method. The term 'consisting of' is used in the same manner as 'composing of' and means to exclude additional elements, steps, or components, which are not separately described. The term "essentially consisting of" means in the scope of the composition or method, including the component elements or steps described, as well as the component elements or steps that do not substantially affect their basic properties.

### [ADVANTAGEOUS EFFECT]

The nutmeg extract of the present invention not only contains a high lignan-based compound, but also, when ingested, the effect of improving athletic ability, improving endurance, and improving muscle strength is very excellent, which is effective in treating sarcopenia, and can be usefully used in medicines and foods for improving physical strength or athletic ability.

### [BRIEF DESCRIPTION OF THE DRAWINGS]

FIG. 1 shows the content of glycogen that appears as a result of treating the nutmeg extract of the present invention to muscle cells.
FIG. 2 shows the weight change of the mice to which the nutmeg extract of the present invention was administered.
FIG. 3 is a graph showing the results of the rotarod test in order to confirm the effect of nutmeg extract on athletic ability and physical strength.
FIG. 4 is a graph showing the results of the rotarod test to confirm the effect of nutmeg extract on athletic ability and physical strength, and more specifically, the results of confirming the athletic ability and endurance of mice in a rod rotating at a low speed to determine the athletic ability according to muscle strengthening.
FIG. 5 is a graph showing the results of the rotarod test in order to confirm the effect of nutmeg extract on athletic ability and physical strength, and more specifically, the results of confirming the athletic ability and endurance of mice in a rod rotating at high speed.
FIG. 6 shows the results of the Wire hang test confirming the effect of improving the athletic ability of muscle strengthening of the old mouse according to the administration of nutmeg extract.
FIG. 7 is a graph measuring the swimming speed by Morris Water Maze test of mice according to the nutmeg extract administration.
FIG. 8 is a graph showing the results of measuring the swimming speed by Morris Water Maze test of the Old mouse group.
FIG. 9 is a result graph showing the maximum swimming duration of a mouse as a result of the forced swim endurance test.
FIG. 10 is a photograph showing changes in the volume of the femur of a mouse as a result of administering a nutmeg extract to a young mouse.
FIG. 11 is a photograph showing changes in the volume of the femur of a mouse as a result of administering a nutmeg extract to an old mouse.
FIG. 12A is a graph showing the results of rotarod testing on mice to which a nutmeg extract was administered to mice, and is a result of confirming the athletic ability and endurance of mice on rods rotating at low speeds.
FIG. 12B is a photograph observed by extracting the liver of a normal mouse and a mouse to which creatine has been administered for a long time.

In the figure, *: p <0.05; **: p <0.01 and ***: p <0.001 indicate a significant difference, respectively.

Abbreviations in the drawings mean:
Y: Young, O: old, V: vehicle; Y-V: Y-vehicle, Y-10: Y-10mg/kg, Y-30: Y-30mg/kg, Y-100: Y-100mg/kg. O-V: O-vehicle, O-10: O-10mg/kg, O-30: O-30mg/kg, O-100: O-100mg/kg.

### [MODE FOR CARRYING OUT INVENTION]

Hereinafter, the present invention will be described in detail.

However, the following examples are illustrative of the present invention, and the present invention is not limited to the following examples.

### Preparation for the experiment

### 1. Plant materials

Dried Myristica fragrans Houtt was collected in Jakarta, Indonesia, and was identified by Dr. Nam-In Paik, Department of Herbal Medicine Ingredients and Manufacturing, Kyunghee University (Yongin, Korea). Evidence samples were deposited with the Department of Biotechnology, Yonsei University (Seoul, Korea).

### 2. Test substances and test systems

C57BL/6 male mice were prepared with 12 mice of 3 months (Young) and 16 mice of 20 months (Old) according to age, and the doses were set to 10, 30, and 100 mg/kg, bw/day. It was approved by JC Pharma Laboratory Animal Ethics Committee in accordance with the ethical regulations of laboratory animals (deliberation number: 17-M001).

The route of administration applies the clinically planned route, and the number and duration of administration were administered once/day, 7 days/week, until the end of the test.

Table 1 describes the test group configuration for in vivo experiments of the present invention.

**Table 1**

| Mo nth age | Test group | Gen der | Numbers of animals | Animal number | Dosage amounts (ml/kg/d ay) | Dosage amounts (mg/kg/d ay) | Days of admin istrati on | Routes of adminis tration |
|---|---|---|---|---|---|---|---|---|
| 3 | Y1 | M | 3 | 1-3 | 4 | 0 | 22 | Oral |
| | Y2 | M | 3 | 4-6 | 4 | 10 | 22 | Oral |
| | Y3 | M | 3 | 7-9 | 4 | 30 | 22 | Oral |
| | Y4 | M | 3 | 10-12 | 4 | 100 | 22 | Oral |
| 20 | O1 | M | 4 | 13-16 | 4 | 0 | 22 | Oral |
| | O2 | M | 4 | 17-20 | 4 | 10 | 22 | Oral |
| | O3 | M | 4 | 21-24 | 4 | 30 | 22 | Oral |
| | O4 | M | 4 | 25-28 | 4 | 100 | 22 | Oral |
| Y1 and O1: negative control (with excipients) | | | | | | | | |
| Y2-Y4 and O2∼O4: Group administrating Test substance | | | | | | | | |

As a test substance, a 30% ethanol extract of nutmeg (Basic Disaster Team, Korea Basic Science Institute) was used, and Polyethylenglycol 400 (PEG 400) (Samchun Pure Chemical Co., Ltd. P0638) was used as an excipient (negative control group).

### 3. Statistical Analysis

For statistical analysis, GraphPad Prism 7 was used and the significance level was set to p<0.05. The measurement values of each test group classified according to the administration concentration were converted into percentages compared to the control group, and an average value (M) and a standard error average (SEM) were used as test results. The difference between each group was statistically analyzed by One-Way or Two-Way ANOVA, and Neuman Keuls Test or Tukey Test was performed by post-test analysis. The difference between each group was analyzed by statistical analysis by One-Way or Two-Way ANOVA, and if significant results were seen, a post-test analysis was performed to evaluate the significance between each group.

### Example 1: Preparation of nutmeg extract

### 1-1. Preparation of nutmeg extract

100g of pulverized nutmeg was added to 500 ml of each solvent (see Table 2) and extracts were prepared for each solvent condition using an ultrasonic extractor three times over two hours. The separated extract was prepared and used at the same concentration, and the myristicin standard was purchased from Sigma (product number: M9237). Each nutmeg solvent extract and myristicin standard were analyzed using HPLC [Optima Pak C18 column 4.6x250mm, 5µm particle size, flow rate 1ml/min, UV detection: 260nm] under MeOH-H2O (0-32min: 63% MeOH, 32-37min : 63→100% MeOH) conditions, and the myristicin content of each nutmeg extract is shown in Table 1.

**Table 2**

| Solvent extraction of alcohol | The content of myristicin in the extract (%) | The content of nectarine B in the extract (%) |
|---|---|---|
| Water extract | 0.19 | 0.62 |
| 10% aqueous ethanol extract | 0.31 | 0.94 |
| 20% aqueous ethanol extract | 0.33 | 2.98 |
| 30% aqueous ethanol extract | 0.45 | 7.48 |
| 40% aqueous ethanol extract | 1.34 | 1.95 |
| 50% aqueous extract using ethanol | 1.48 | 2.77 |
| 75% aqueous ethanol extract | 1.27 | 2.95 |
| 75% aqueous methanol extract | 1.85 | 7.47 |

As shown in Table 2, it was confirmed that the myristicin content of the 30% aqueous ethanol extract is extracted on average about three times less than that of the other conditions. In addition, as a result of analyzing the content of the nectarine B using the HPLC [OptimaPak_C18 column 4.6x250mm, 5µm particle size, flow rate 1ml/min, UV detection: 205, 280nm] of MeOH-H2O (0-35min: 60% MeOH, 35-60min: 60→100% MeOH) for the nutmeg solvent extract under each condition, it was found that the nectarine B was most extracted from the 30% aqueous ethanol extract. Therefore, it was confirmed that the condition for minimizing the content of toxic substances while maximizing the amount of the active substance is extracting nutmeg with an aqueous ethanol solution of 30% or less.

### 1-2. Identification of compounds extracted from nutmeg

2,5-bis-aryl-3,4-dimethyltetrahydro furan lignan-based compound was isolated using HPLC in the nutmeg extract of a 30% aqueous ethanol solution of the Example 1.

As a result, tetrahydrofuroguaiacin, Saucernetindiol, Verrucosin, Nectandrin B, Nectandrin A, and Fragransin C1, a total of 6 compounds were isolated.

### Example 2: Measurement of weight change in mice

Body weights were measured before administration (when taken and separated) and from the start of administration to the end of the experiment. As a positive control, creatine (120 mg/kg) was administered.

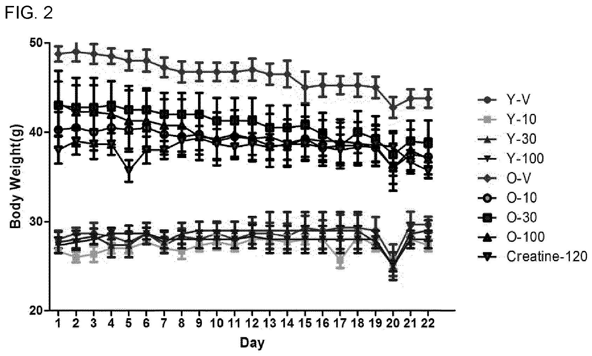

As shown in FIG. 2 the weight change of the group to which the nutmeg extract was administered was similar to that of the excipient control group, and the weight change was the same in the tested Young (Y) and Old (O) age groups.

A temporary weight loss was observed in all test groups on the 20th day of administration, which was judged to be due to the result of discontinuing the dietary supply one day prior to performing the neurological deficit test by administration to the nutmeg extract. It showed that the weight had been recovered by supply.

### Example 3: Measurement of glycogen content in muscle cells (in vitro)

C2C12 Cell was resuspended in a 25 cm2 flask using Growth Medium containing 10% fetal bovine serum, Penicillin-Streptomycin-Glutamine, and 50µl Sodium Pyruvate added to Dulbecco's Modified Eagle's Medium. The growth medium was changed after 6 hours. After maintaining the 50-70% confluency, the cells were cultured and subcultured with 100 X 20mm dish. At that time, the cell concentration was 1:13-15 dilutions. The cells were seedling at a concentration of 2.5 x 105/6 well in a 6 well plate. When it was 90% confluency, the medium was changed to a differentiation medium containing 2% Horse Serum and Penicillin-Streptomycin-Glutamine in Dulbecco's Modified Eagle's Medium. The medium was changed to a differentiation medium every 24 hours (for 5 days). The differentiation under a microscope (make sure to differentiate and form a myotube) was confirmed. After the differentiation was completed, the extract or indicator was treated.

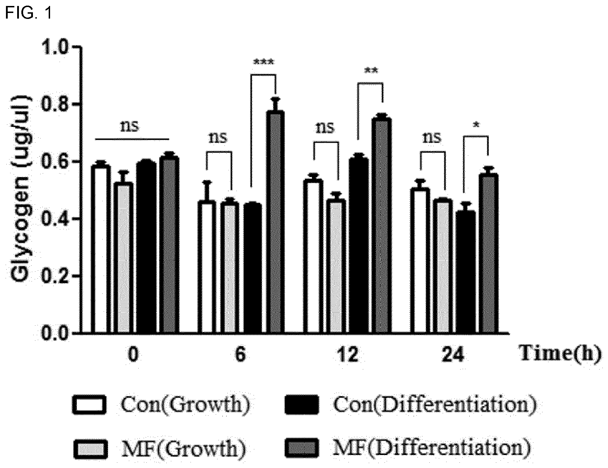

The glycogen content of the cells was measured using a Glycogen assay kit (Biovision). Specifically, the C2C12 muscle cells from which the culture solution was removed were washed twice with cold PBS, and then the plate was placed on ice, 200 µl of distilled water per well was added, scraped, placed in an ep-tube, and then lysed with an ultrasonicator. Using 96-well plates, 50 µl and 2 µl hydrolysis enzyme were respectively added to the nutmeg extract sample (10mg/kg/day, 20mg/kg/day) and the standard solution in a well, and reacted at room temperature for 30 minutes. After completion of the reaction for 30 minutes, 50 µl of the reaction mixture was dispensed, and then reacted for 30 minutes at room temperature at a shade, and absorbance was measured at 570 nm. The results were shown in FIG. 1.

As a result, it was confirmed that the content of glycogen in the differentiated muscle cells was significantly increased according to the nutmeg extract treatment as shown in FIG. 1. This can be said that the nutmeg extract of the present invention is a direct basis for indicating endurance exercise capacity.

### Example 4: Rotarod test

The equipment used for the test was a rotarod from SB Technology, and was tested in a fixed mode and an acceleration mode rotating at a speed of 4 or 4-40 rpm with a rotarod equipped with a rod for a mouse. The mice to be tested 1 day before the start of the test, 7 days after administration, were adapted in the laboratory for 1 hour, and then pre-training was performed for 1 minute at a fixed 4 rpm condition. At this time, the mouse that fell within 1 minute was put on the rod again, and trained on the rod for a total time of 1 minute. Before starting the test, the test was conducted after being adapted to the test environment for at least 30 minutes, and the test was conducted for 5 minutes in an accelerated mode rotating at a speed of 4-40 rpm per mouse individual. And the time of falling moment on the rod was recorded.

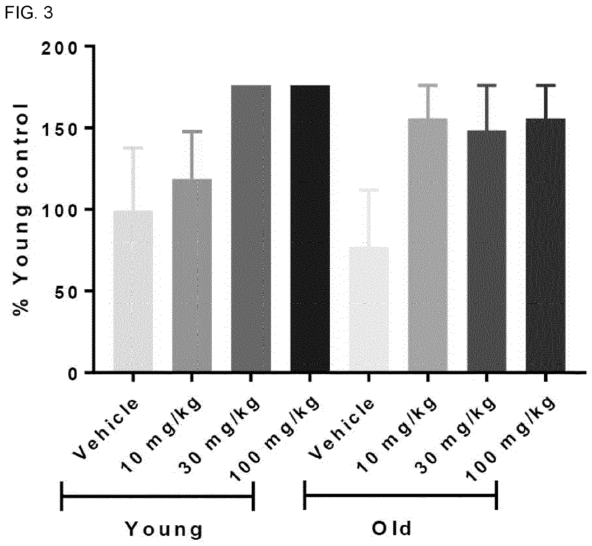

As a result, as shown in FIGS. 3 to 5, it was confirmed that the athletic ability and endurance of the mouse significantly increased since the nutmeg methanol extract has been treated.

As shown in FIG. 3, Young and Old mice not administered with nutmeg extract showed significantly lower the athletic ability and endurance compared to the Young and the Old mice administered with nutmeg extract in rotarod rotating at 4 rpm.

In other words, Young and Old mice administered with nutmeg extract showed superior athletic ability and endurance compared to mice without nutmeg extract, despite no concentration-dependent experience in rotarod.

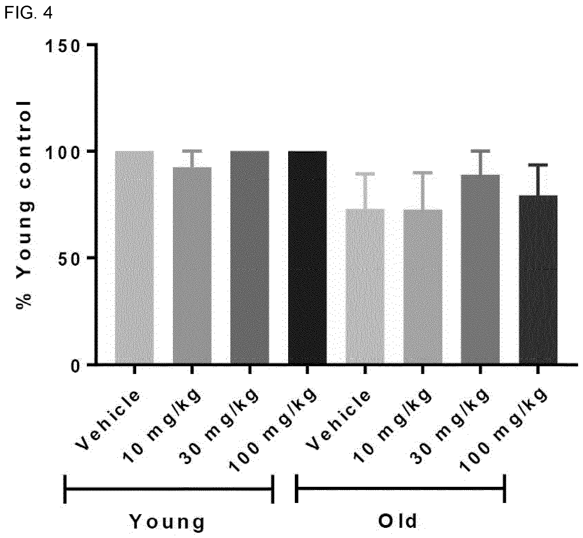

As shown in FIG. 4, in order to determine the athletic ability according to muscle strengthening by the administration of nutmeg extract, the criterion showing that both Young and Old mice showed similar athletic ability regardless of whether or not the test substance was administered in a rod rotating at a low speed was found in training continuously at a low speed of 4 rpm.

It can be confirmed from FIG. 4 that after 3 days of training, the conditions for the movement and endurance of all mice were secured (F(7,20)=0.8985, p=0.5265).

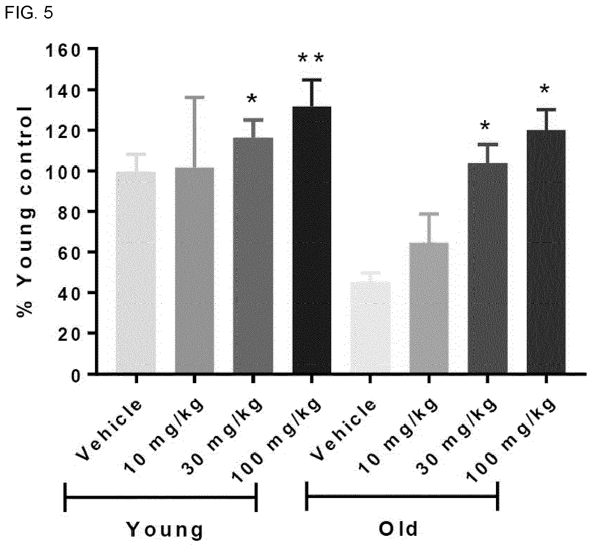

As shown in FIG. 5, as a result of testing in the accelerated mode of rotarod, the athletic ability and endurance were improved in a concentration-dependent manner in both Young and Old mice by administration of nutmeg extract (F(7,19)=4.3180, p= 0.0052). In Young mice, the improvement of athletic ability and the increase of endurance with concentration of nutmeg extract showed concentration-dependent effects, but there was no statistical significance (F(3,8)=0.5841, p=0.6421). In the case of Old mice, the concentration-dependent effect of nutmeg extract showed statistical significance (F(3,11)=10.79, p=0.0013).

In conclusion, it was shown that the old mice dosed with 30 and 100 mg/kg, bw showed statistically significant improvement in athletic ability and endurance of 2.3 times and 2.6 times, respectively, compared to old mice without nutmeg extract (p<0.01). Old mice dosed with 10 mg/kg, bw showed an increase in athletic ability and endurance of 1.4 times. Particularly, in the case of the old mouse to which 30 and 100 mg/kg, bw of the nutmeg extract were administered, it showed similar athletic ability and endurance as the Young mouse, and thus it was confirmed that the nutmeg extract of the present invention is effective in improving exercise capacity and endurance.

The statistically significant improvement in concentration-dependent athletic ability in the old mouse according to nutmeg extract means that it has the same effect as that in the Young mouse by restoring the athletic ability of the old mouse with reduced exercise ability due to muscle weakness. In addition, nutmeg extract suggests that the results of improving the motor skills of Young mice may strengthen the normal muscle strength.

### Example 5: Wire hang test

The test was performing using a wire having a diameter of 2 mm installed at a height of 25 cm. Each mouse was placed on the wire using only the front legs, and the time of hanging without falling was measured. The time when the mouse was suspended and then dropped was measured and the test was conducted for a maximum of 120 seconds. In this way, each mouse was repeated 3 times at approximately 20 minute intervals. The results of each mouse were analyzed for the average value of the first and longest hanging time in three trials. All Young mice in the nutmeg extract-administered group and the excipient control group were tested only in the Old mice because they were not suitable for evaluating the exercise ability of the pure forelimbs only because they showed the behavior of pulling the wire by the muscles of the strong forelimbs and pulling up the wire with the hind limbs. In addition, creatine 120 mg/kg (creatine-120) was administered to mice as a positive control, and the test was conducted under the same conditions.

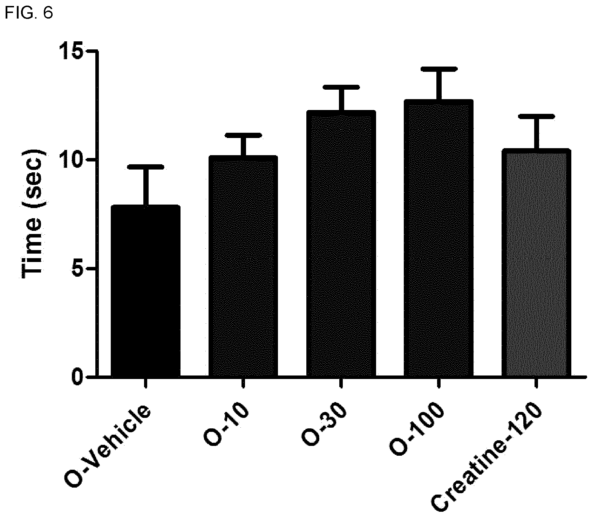

As shown in FIG. 6, as a result, it was found that the improvement of athletic ability and endurance according to muscle strengthening was statistically significantly increased depending on the concentration of nutmeg extract administration (F(3,11)=4.555, p=0.0262). The effect of enhancing athletic ability by muscle strengthening by administration of nutmeg extract was increased by 25%, 58% and 97% at 10, 30 and 100 mg/kg, bw, respectively and the administration group of 100 mg/kg, bw showed a statistically significant increase effect (p<0.05).

In addition, when compared with the results of the positive control group (creatine-120), it was confirmed that it was more effective than the aging mouse group, but when the nutmeg extract 10mg/kg was administered, the effect of improving motor function was similar. On the other hand, when 30mg/kg and 100mg/kg of nutmeg extract respectively were administered, it was confirmed that they showed a remarkable effect on improving motor function compared to the positive control group.

Through these results, it means that nutmeg extract can strengthen the muscles of the fore limbs of aged or degenerated Old mice, which can improve athletic ability and endurance, suggesting that these effects are non-specifically involved in exercise-related muscles.

### Example 6: Morris water maze test

Morris water maze placed mice swimming in a circular pool with a featureless interior surface in a room with walls with various visible external markers attached. The water temperature in the water labyrinth was adjusted to room temperature or 21°C, and skim milk powder was mixed to make the water labyrinth surface opaque, and the only platform that could escape the water in the labyrinth was placed in one of the four quadrants of the round labyrinth. The water labyrinth test was performed with a visible version where the pretraining and platform can be seen from the surface. Pretraining allowed all mice to swim freely in a water labyrinth without platforms for 60 seconds, and the swimming speed of each mouse was used as an indicator of athletic ability.

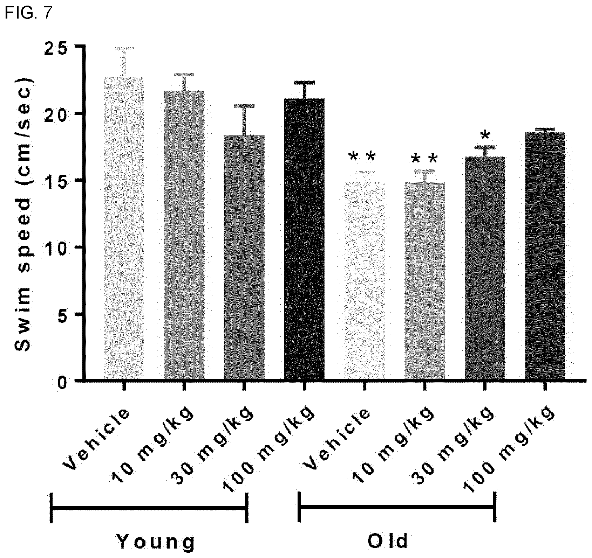

As shown in FIG. 7, in the Young mice, swimming speed was consistent in all groups regardless of the nutmeg extract administration. In the Old mouse, it showed a tendency to improve swimming speed by nutmeg extract administration statistically significantly (F(7,20)=6.647, p=0.0004).

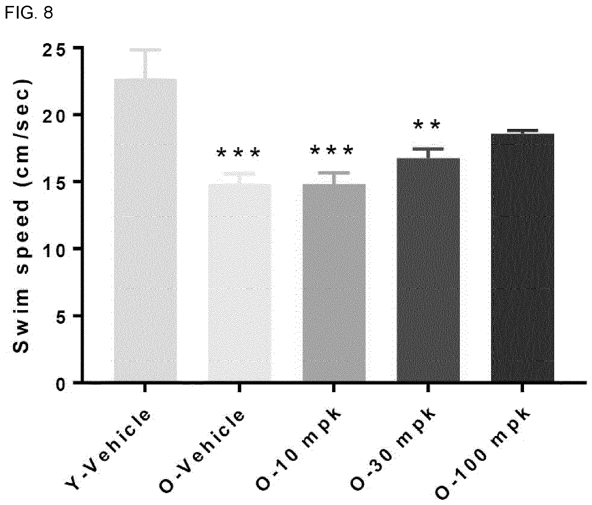

As shown in FIG. 8, the same tendency was observed when only the Young and Old mice, which were not treated with nutmeg extract, were analyzed statistically (F(4,14)=10.23, p=0.0004).

### Example 7: Neurological deficit test

To determine the presence or absence of neurological deficit according to the administration of a nutmeg extract, rotarod fixed at 6 rpm was used.

In order to exclude interactions with ingested food, all experimental animals were fasted for 18 hours, and nutmeg extract was administered at 100, 300, and 1,000 mg/kg, and then the number of experimental animals falling from the rotarod for 1 minute at 0.5, 1 and 2 hours was recorded.

As a result, as shown in Table 3, 1,000 mg/kg, bw of nutmeg extract was administered to each of Young and Old mice and tested by time period. As a result, all mice tested did not drop successfully from the rotarod.

In the Old mice, at 0.5 and 1 hour after administration of the test substance, all mice did not fall under the tested conditions, but at 2 hours, only one was not satisfied with the conditions.

**Table 3**

| | Test time (number of mice fallen/number of mice tested) | | |
|---|---|---|---|
| | 0.5 hr | 1 hr | 2 hr |
| Young-control | 3/3 | 2/3 | 3/3 |
| Young-1000 mg/kg, bw | 3/3 | 3/3 | 3/3 |
| Old-control | 2/4 | 4/4 | 2/4 |
| Old-1000 mg/kg, bw | 4/4 | 4/4 | 3/4 |

In addition, the test result of Neurologic deficit means that there is no effect on the nervous system according to the administration of nutmeg extract. That is, the nutmeg extract is estimated to have a TD50 value of 1,000 mg/kg, bw or more, with 50% toxic dose that shows neurological deficit, and at the same time, based on the results of the rotarod test, when predicted to be at least 10 to 30 mg/kg at a concentration of 50% of nutmeg to show the athletic ability of the Young mice, the protective index (PI) value is judged to be at least 34, suggesting that it is a very safe material.

### Example 8: Forced swim endurance test

The forced swim endurance test was conducted by modifying Jacob and Michud's method (Jacob J, Michud G. Action of various pharmacological agents on the exhaustion time and behavior of mice swimming at 20 degree C. I. Description of the technic. Actions of amphetamine, cocaine, caffeine, hexobarbital and merprobamate. Arch. Int. Pharmacodyn. Ther. (1961) 133: 101.). A circular water tank with a diameter of 120 cm was filled with water to a depth of 30 cm and the water temperature was maintained at 21±1°C. A weight about 5% of the weight of each mouse was hung on the tail, and 3-4 mice from each group are put into the tank at the same time. The time until swimming and exhaustion was measured. The test was performed within a limited time of 5 minutes.

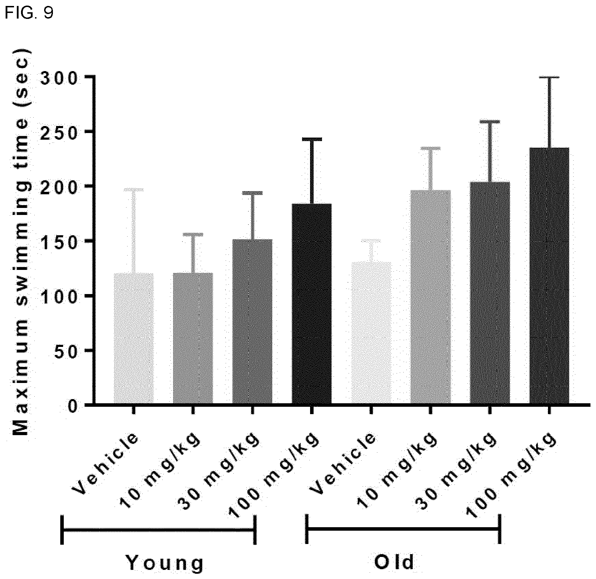

As a result, as shown in FIG. 9, both Young and Old mice administered with the nutmeg extract showed a tendency to increase swimming endurance in a concentration-dependent manner. During the forced swim endurance test, the Young mice showed more activity than the Old mice, and few inappropriate floating behaviors were observed compared to the Old mice.

### Example 9: The effect of increasing the muscle mass of nutmeg extract

Three and 10-month C57BL/6 mice were randomly divided into 8 groups of 3 animals, respectively, according to Table 1, and used in the experiment.

In the experimental group, 30% ethanol extract of nutmeg was administered at a constant time for a total of 22 days once a day at three concentrations of 10 mg/kg, 30 mg/kg, and 100 mg/kg. At this time, as a control, polyethylene glycol as an excipient was used. After the end of the test, the rat's thigh was separated and the volume was measured.

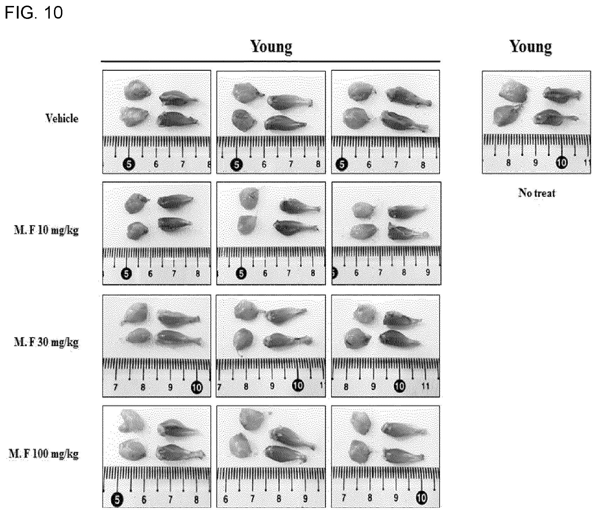

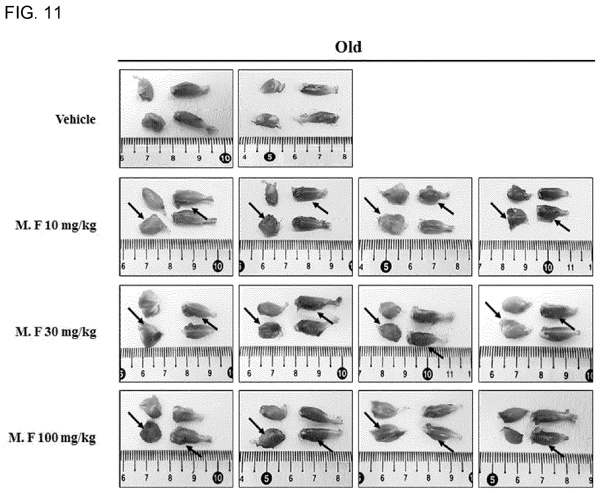

As a result, as shown in FIGS. 10 and 11, compared to the control group, the muscle mass and volume increased overall in the group administered with the nutmeg extract regardless of the age of the rat, especially in the Old mouse. As a result, it was confirmed that the nutmeg extract of the present invention increased the muscle mass, and thus, the effects of the athletic ability and muscle strength improvement were very excellent.

### Example 10: Rotarod test and liver toxicity confirmation

The rotarod test was performed under the same conditions and methods as in Example 4. At this time, as a positive control, mice administered with creatine 120 mg/kg (creatine-120) were used. Specifically, the mice to be tested 1 day before the start of the test, 7 days after administration, were adapted in the laboratory for 1 hour, and then pre-training was performed for 1 minute at a fixed 4 rpm condition. At this time, the mouse that fell within 1 minute was put on the rod again, and trained on the rod for a total time of 1 minute. Before starting the test, the test was performed after being adapted to the test environment for at least 30 minutes, and the test was conducted in an accelerated mode rotating at a speed of 4-40 rpm per mouse. And the time of falling moment on the rod was recorded.

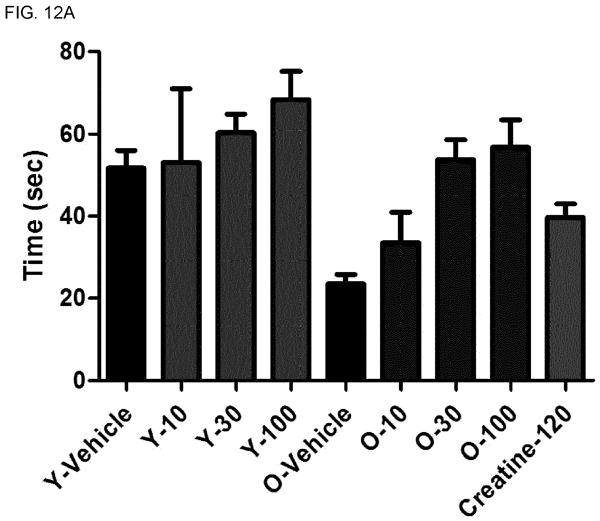

As a result, as shown in FIG. 12A, the effect of exercise improvement was similar when creatine and 10 mg/kg of nutmeg extract was administered. On the other hand, when the nutmeg extract was administered at a concentration of 30mg/kg, and 100mg/kg, it was confirmed that the effect of exercise improvement was remarkably higher than when the creatine was administered.

And then, experiments were performed as follows to confirm the effect of nutmeg extract on the liver.

After the acclimatization of the three-month-old mouse, each group was divided into a group that was orally administered with creatine 120 mg/kg for 22 days, a group that was orally administered with nutmeg extract 100 mg/kg for 22 days, and a group that had only normal diet for 22 days. Then, the liver was removed and observed at the expense of each mouse.

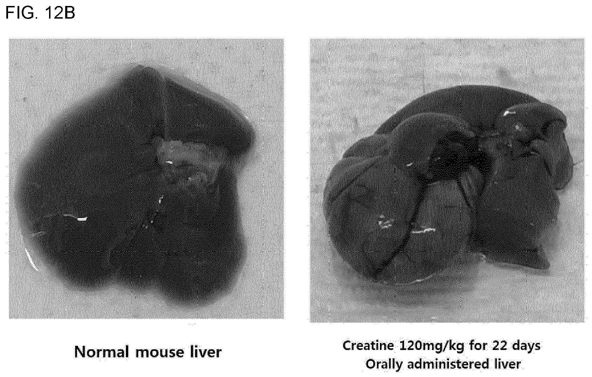

As a result, as shown in FIG. 12B, when creatine was administered, it was confirmed that the liver lobe was enlarged, and it was confirmed that liver toxicity occurred during long-term use. On the other hand, when the nutmeg extract was administered, the liver lobe was not enlarged, and the shape of the liver was found to be the same as that of a normal mouse (data not shown).

Therefore, it was confirmed that nutmeg extract did not show liver toxicity even after long-term use.

### [INDUSTRIAL APPLICABILITY]

As discussed above, the nutmeg extract of the present invention contains a high quantity of ligand-based compounds and exhibits, when taken, very excellent effects of improving athletic ability, improving endurance and improving muscular strength, and thus is effective for treating sarcopenia and can be effectively used in medical supplies, food and the like for improving physical strength or athletic ability.

## Claims

1. A food composition for improving physical strength or athletic ability, comprising a nutmeg extract as an active ingredient.

2. The composition of claim 1, wherein the nutmeg extract is a pharmaceutical composition, extracted with one or more solvents selected from the group consisting of water, alcohol having 1 to 6 carbon atoms, acetone, ether, benzene, chloroform, ethyl acetate, methylene chloride, hexane, cyclohexane, petroleum ether, subcritical fluid and supercritical fluid.

3. The composition of claim 1, wherein the improvement in athletic ability is to increase muscle endurance or strengthen muscle strength.

4. The composition of claim 1, wherein the nutmeg extract further comprises a property that increases the level of glycogen at a cellular level.

5. The composition of claim 1, wherein the composition is for a middle-aged or older individual.

6. A pharmaceutical composition for improving physical strength or athletic ability, comprising a nutmeg extract as an active ingredient.

7. A pharmaceutical composition for preventing or treating sarcopenia comprising a nutmeg extract as an active ingredient.

8. A food composition for preventing or improving sarcopenia comprising nutmeg extract as an active ingredient.

9. A food composition for improving muscle function comprising the nutmeg extract as an active ingredient.

10. A food composition for anti-fatigue comprising a nutmeg extract as an active ingredient.

11. A composition as a feed additive for improving physical strength, improving athletic ability, or increasing the amount of muscle comprising the nutmeg extract as an active ingredient.

12. Use of a nutmeg extract to prepare an agent for improving physical strength or improving athletic ability.

13. A method for improving physical strength or athletic ability in a subject, the method comprising administering an effective amount of a composition comprising a nutmeg extract as an active ingredient to the subject in need thereof.

14. Use of nutmeg extract to prepare an agent for preventing or improving sarcopenia.

15. A method for preventing or treating sarcopenia in a subject, the method comprising administering an effective amount of a composition comprising a nutmeg extract as an active ingredient to the subject in need thereof.

16. Use of nutmeg extract to prepare an agent for improving muscle function.

17. A method for improving muscle function, the method comprising administering an effective amount of a composition comprising a nutmeg extract as an active ingredient to a subject in need thereof.

18. Use of nutmeg extract to prepare an anti-fatigue agent.

19. An anti-fatigue method, the method comprising administering an effective amount of a composition comprising a nutmeg extract as an active ingredient to a subject in need thereof.
